# EUROPÄISCHE PATENTSCHRIFT

(11) **EP 1 019 354 B1**
(45) Veröffentlichungstag und Bekanntmachung des Hinweises auf die Patenterteilung: **05.12.2001**
(21) Anmeldenummer: 98950060.8
(22) Anmeldetag: 19.09.1998
(51) Int. Cl.: C07C 45/50, B01J 31/22, B01J 31/24, B01J 31/28

(54) **VERFAHREN ZUR HERSTELLUNG VON ALDEHYDEN**
METHOD FOR PRODUCING ALDEHYDES
PROCEDE POUR PRODUIRE DES ALDEHYDES

(30) Priorität: 29.09.1997 DE 19742907
(43) Veröffentlichungstag der Anmeldung: 19.07.2000
(73) Patentinhaber: Celanese Chemicals Europe GmbH, 60439 Frankfurt am Main (DE)
(72) Erfinder: BAHRMANN, Helmut, D-46499 Hamminkeln (DE); SALZER, Albrecht, D-52074 Aachen (DE); BRASSE, Claudia, D-52074 Aachen (DE)
(86) Internationale Anmeldenummer: EP9805988
(87) Internationale Veröffentlichungsnummer: WO9916737

(56) Entgegenhaltungen:
- WO-A-97/08124
- DE-A- 4 040 315
- US-A- 4 169 861
- US-A- 4 221 744
- RUDIE A W ET AL: "Comparative study of 1,1'-bis(diphenylphosphino)cobaltocinium hexafluorophosphate and 1,1'-bis(diphenylphosphino)ferrocene as bidentate ligands" INORG. CHEM. (INOCAJ,00201669);78; VOL.17 (10); PP.2859-63, XP002089110 Massachusetts Inst. Technol.;Dep. Chem.; Cambridge; Mass.
- CHEMICAL ABSTRACTS, vol. 112, no. 11, 12. März 1990 Columbus, Ohio, US; abstract no. 097991, MAGOMEDOV G K ET AL: "Catalysis of hydroformylation of 1-octene by cyclopentadienylcobalt compounds" XP000061083 & METALLOORG. KHIM. (MEKHEX);89; VOL.2 (4); PP.806-7, Gos. Inst. Khim. Tekhnol. Elementoorg. Soedin.;Moscow; USSR (SU)
- Lorkovic, I.M.; Duff, R.R.; Wrighton, M.S. "Use of the Redox-Active Ligand 1,1'- Bis(diphenylphosphino)cobaltocene to Reversibly Alter the Rate of the Rhodium(I)-Catalyzed Reduction and Isomerization of Ketones and Alkenes", J. Amer. Chem. Soc. 1995, vol. 117, no. 12, pages 3617-8
- Lorkovic, I.M.; Wrighton, M.S.; Davis, W.M. "Use of a Redox-Active Ligand to Reversibly Alter Metal Carbonyl Electrophilicity", Journal of the American Chemical Society 1994, vol. 116, no. 14, pages 6220-8

## Beschreibung

Aldehyde besitzen als wertvolle Zwischenprodukte eine große wirtschaftliche Bedeutung. Aus ihnen lassen sich z.B. Alkohole, Carbonsäuren und Amine herstellen, die wiederum als Ausgangsprodukte für die Herstellung wichtiger Endprodukte verwendet werden.

Es ist bekannt, durch Umsetzung von Olefinen mit Kohlenmonoxid und Wasserstoff Aldehyde herzustellen. Die Reaktion wird durch Hydridometallcarbonyle, vorzugsweise solcher der Metalle der Gruppe VIII des Periodensystems der Elemente, katalysiert. Während zunächst Kobalt als Katalysatormetall in großem Umfang technische Anwendung fand, haben sich Verfahren zur Herstellung von Aldehyden, die Rhodium als Katalysatormetall verwenden, in der Zwischenzeit in der Technik etabliert.

Das unter Rhodiumkatalyse durchgeführte Hydroformylierungsverfahren zeichnet sich durch ein im Vergleich zum Kobaltverfahren höheres Verhältnis an geradkettigen zu verzweigten Aldehyden (n/iso-Verhältnis) aus. Ein möglichst hoher Gehalt an n-Aldehyden ist erwünscht.

Die Herstellung von Aldehyden kann einphasig in einer organischen Phase erfolgen. Dabei liegt der Katalysator, wie z.B. ein Rhodium/Triphenylphosphinkomplex, gelöst in dem organischen Reaktionsgemisch vor. Darüber hinaus kann die Herstellung von Aldehyden auch in Gegenwart eines organischen Lösungsmittels erfolgen. Als Lösungsmittel werden z.B. Toluol, Xylol oder Tetrahydrofuran verwendet.

Aus US 4,169,861 ist bekannt, daß sich 1,1'-Bis-(Diphenylphosphino)ferrocen als zweizähniger Chelatligand in Rhodiumkomplexen verwenden läßt, die als Katalysator für Hydroformylierungsreaktionen eingesetzt werden. Nach dem bekannten Verfahren erfolgt die Herstellung von Aldehyden durch Reaktion von alpha-Olefinen, vorzugsweise 1-Hexen, mit Kohlenmonoxid und Wasserstoff bei Temperaturen zwischen 25 °C und 150 °C und bei einem Druck von 15 bis 3000 psi (dies entspricht 1,03 bis 207 MPa), bevorzugt bei einem Druck von 50 - 500 psi (dies entspricht 3,45 bis 34,5 MPa)einphasig in Gegenwart eines Rhodiumkatalysators, der, bezogen auf ein Grammatom Rhodium, ein Mol 1, 1'-Bis(diphenylphosphino)ferrocen enthält. Neben den Phenylgruppen können an den Phosphoratomen auch Alkylgruppen, Arylgruppen, alkoxy-substituierte Arylgruppen und Fluor-substituierte Arylgruppen mit bis zu 20 Kohlenstoffatomen gebunden sein. Darüber hinaus wird ein 5 bis 100 facher molarer Überschuß eines einzähnigen Liganden verwendet, bei dem es sich um ein tertiäres Amin, Phosphin oder Arsin handelt. Der einzähnige Ligand trägt aliphatische, alicyclische oder aromatische Gruppen mit 1 bis 20 Kohlenstoffatomen. Das hier beschriebene Hydroformylierungsverfahren wird einphasig in Toluol als Lösungsmittel durchgeführt und liefert n-Heptanal und 2-Methylhexanal in einem Verhältnis von 5:1.

Auch US-A-4,221,744 offenbart ein Hydroformylierungsverfahren in Gegenwart Rhodium haltiger Komplexverbindungen, die als Liganden das 1,1'-Bis-(Diphenylphosphino)ferrocen enthalten können, wobei die Diphenylphosphinogruppe noch zusätzlich elektronenziehende Substituenten, wie z.B, die Methoxy-, die Trifluormethylgruppe oder Fluor enthält. Nach der Lehre von US-A-4,221,744 führt die Anwesenheit solch elektronenziehender Substituenten im Ferrocenliganden zu einer Erhöhung des Verhältnisses von geradkettigen zu verzweigten Aldehyden in der Hydroformylierungsreaktion.

In der Technik ist ein hoher Gehalt an n-Aldehyden gewünscht.

Die Verwendung des ungeladenen 1,1'-Bis(diphenylphosphino)cobaltocen und des einfach geladenen 1,1'-Bis(diphenylphosphino)cobalticinium-Kations als Ligand in Rhodiumkomplexen, die katalytische Eigenschaften aufweisen, ist aus J. Am. Chem. Soc. 1995 117, 3617-3618 bekannt. In Aceton als Lösungsmittel erhält man unter Verwendung von Bis(diphenylphoshino)cobaltocen einen einfach geladenen zweikernigen Rhodiumkomplex, der sich als Hydrierkatalysator von Olefinen und Ketonen sowie als Olefinisomerisierungs-Katalysator bewährt. Setzt man jedoch das 1,1'-Bis(diphenylphosphino)cobalticinium-Kation ein, erhält man einen zweifach geladenen Rhodiumkomplex, der als Silylierungskatalysator von Olefinen oder Ketonen wirkt.Durch die Wahl des Redox-Zustandes können daher die Katalysatoreigenschaften eingestellt werden.

Nach J. Am. Chem. Soc. 1994, 116, 6220-6228 läßt sich die Reaktivität von Rheniumcarbonylen gezielt einstellen, wenn als Ligand 1,1'-Bis(diphenylphosphino)cobaltocen verwendet wird, der wiederum in das 1,1'-Bis(diphenylphosphino)cobalticinium Kation oxidiert werden kann. Als Folge der Oxidation des Kobalts werden die Carbonylkohlenstoffatome in den Rheniumcarbonylen für den Angriff von Nukleophilen, wie z.B. von Aziden oder Aminoxiden, zugänglicher.

Aus der Deutschen Offenlegungsschrift DE 4040315 ist ein Verfahren zur Herstellung von Aldehyden bekannt, bei dem sulfonierte Diphosphane, z.B. sulfoniertes 2,2'-Bis(diphenylphosphino)-biphenyl, als Liganden verwendet werden. Derartige Diphosphane sind jedoch aufwendig herzustellen und erhöhen daher die Kosten für das eingesetzte Katalysatorsystem.

Vor dem Hintergrund, daß die Hydroformylierungsreaktionen in Gegenwart von katalytisch wirksamen Metallkomplexen durchgeführt werden, bestand daher die Aufgabe ein weiteres Verfahren für die Herstellung von Aldehyden zu entwickeln, das die gewünschten geradkettigen Aldehyde in einem hohen Anteil liefert, um so das Spektrum der verfügbaren Verfahren für die Herstellung von Aldehyden zu erweitern.

Die Erfindung besteht in einem Verfahren zur Herstellung von Aldehyden durch Umsetzung von Monoolefinen, nichtkonjugierten Polyolefinen, Cycloolefinen oder Derivaten dieser Verbindungsklasse mit Kohlenmonoxid und Wasserstoff bei Temperaturen von 20 bis 150°C und Drücken von 0,1 bis 20 MPa in Gegenwart einer organischen Lösung als Katalysator, die mindestens eine Rhodiumverbindung und mindestens ein phosphansubstituiertes Cobalticiniumsalz enthält. Es ist dadurch gekennzeichnet, daß phosphansubstituierte Cobalticiniumsalze der allgemeinen Formel I 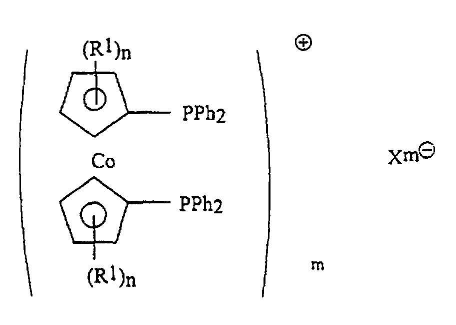 verwendet werden, wobei R¹ gleich oder verschieden ist und Wasserstoff, Alkyl- oder Alkoxyreste mit 1 bis 14 Kohlenstoffatomen, ferner Cycloalkyl-, Aryl- oder Aroxyreste mit 6 bis 14 Kohlenstoffatomen, einen anellierten Benzolring, einen Säureamidrest -C(O) -NR²R³, in dem R² und R³ gleich oder verschieden sind und für Wasserstoff, Alkylreste mit 1 bis 14 Kohlenstoffatomen, Cycloalkyl-oder Arylreste mit 6 bis 14 Kohlenstoffatomen stehen, die Carboxylgruppe oder einen Esterrest -C(O)-OR⁴, in dem R⁴ für einen Alkylrest mit 1 bis 14 Kohlenstoffatomen, ferner für einen Cycloalkyl- oder Arylrest mit 6 bis 14 Kohlenstoffatomen steht, bedeutet, n gleich oder verschieden ist und eine ganze Zahl von 0 bis 4 ist, m eine ganze Zahl von 1 bis 3 ist und X gleich Hexafluorophosphat, Hexafluoroantimonat, Tetraphenylborat, Tosylat, Tetrafluoroborat, Triflat, Trifluoracetat, Triiodid, Tribromid, Nitrat, Perchlorat, Sulfat, Phosphat, Sulfonat ⁻O-S(O)₂-R⁵, wobei R⁵ einen Alkylrest mit 1 bis 14 Kohlenstoffatomen, Cycloalkyl- oder Arylrest mit 6 bis 14 Kohlenstoffatomen bedeutet, oder Carboxylat ⁻O-C(O)-R⁶, wobei R⁶ für Wasserstoff, einen Alkylrest mit 1 bis 14 Kohlenstoffatomen, ferner für einen Cycloalkyl- oder Arylrest mit 6 bis 14 Kohlenstoffatomen steht, ist.

Die Herstellung der phosphansubstituierten Cobalticiniumsalze der allgemeinen Formel I ist Gegenstand der PCT/EP98/05989 (WO-A-9916776).

Nach dem erfindungsgemäßen Verfahren lassen sich Monoolefine , nicht konjugierte Polyolefine, cyclische Olefine und Derivate dieser ungesättigten Verbindungen umsetzen. Die Olefine können geradkettig oder verzweigt, die Doppelbindungen end- oder innenständig sein. Beispiele für Olefine, die in dem neuen Verfahren verwendet werden können, sind Ethylen, Propylen, Buten-1, Buten-2, Penten-1, 2-Methylbuten-1, Hexen-1, Hexen-2, Hepten-1, Octen-1, Octen-3, 3-Ethylhexen-1, Decen-1, Undecen-3, 4,4-Dimethylnonen-1,, Dicyclopentadien, Vinylcyclohexen, Cyclooctadien, Styrol. Derivate der genannten Olefinarten, die nach der beanspruchten Arbeitsweise hydroformyliert werden können sind z.B. Alkohole, Aldehyde, Carbonsäuren, Ester, Nitrile und Halogenverbindungen, Allylalkohol, Acrolein, Methacrolein, Crotonaldehyd, Methylacrylat, Ethylcrotonat, Diethylfumarat und Diethylmaleinat, Acryl nitril. Mit besonderen Erfolg wird das Verfahren zur Hydroformylierung von Olefinen und Olefinderivaten mit 2 bis 12 Kohlenstoffatomen eingesetzt.

Die Herstellung der Aldehyde erfolgt in Gegenwart einer organischen Lösung als Katalysator, die mindestens eine Rhodiumverbindung und mindestens ein phosphansubstituiertes Cobalticiniumsalz der allgemeinen Formel I enthält. Dabei ist anzunehmen, daß sich in Gegenwart von Synthesegas aus dem phosphansubstituierten Cobalticiniumsalz und der eingesetzten Rhodiumverbindung unter den Bedingungen der Hydroformylierungsreaktion katalytisch wirksame Rhodium-Komplexverbindungen bilden, die Kohlenmonoxid und das phosphansubstituierte Cobalticiniumkation als Liganden enthalten und in der organischen Phase gelöst vorliegen. Die in Gegenwart von Synthesegas gebildeten, das phospansubstituierte Cobalticiniumkation enthaltenden Rhodiumverbindungen sind kationischer Natur, die durch das Anion X aus dem phosphansubstituierten Cobalticinium-salz abgesättigt werden.

Als organische Phase läßt sich das Reaktionsgemisch, bestehend aus dem eingesetzten Olefin und dem bereits gebildeten Aldehyd ohne und mit Lösungsmittelzusatz verwenden. Arbeitet man mit Lösungsmittelzusatz, wird bevorzugt Toluol, Xylol, Tetrahydrofuran oder Aceton verwendet.

Bevorzugte phosphansubstituierte Cobalticiniumsalze der Formel I sind solche, in denen R¹ gleich oder verschieden ist und für Wasserstoff, für einen Methyl-, Isopropyl-, Isobutyl-, t-Butyl, Phenyl- oder Naphthylrest oder einen anellierten Benzolring (so daß sich eine Indenylstruktur ausbildet) steht, n gleich 0 oder 1 ist, m gleich 1 oder 2 ist und X für Tetrafluoroborat, Nitrat oder Sulfat steht.

Wie in PCT/EP98/05989 (WO-A-9916776) offenbart, ist 1,1'-Bis(diphenylphosphino)cobalticinium Nitrat in Wasser löslich. Eine wäßrige Lösung von 1,1'-Bis(diphenylphosphino)cobalticinium Nitrat eignet sich in Gegenwart einer Rhodiumverbindung zur Herstellung einer wäßrigen Katalysatorlösung, die in einem Zweiphasen-Hydroformylierungsprozeß zur Herstellung von Aldehyden durch Umsetzung von Monoolefinen, nichtkonjugierten Polyolefinen, Cycloolefinen oder Derivaten dieser Verbindungsklasse mit Kohlenmonoxid und Wasserstoff bei Temperaturen von 20 bis 150°C und Drücken von 0,1 bis 20 MPa eingesetzt werden kann. Ein solcher Zweiphasenprozeß ist durch das Vorliegen einer organischen, Ausgangsolefin und gewünschten Aldehyd enthaltenden Phase und durch das Vorliegen einer wäßrigen, den Hydroformylierungskatalysator enthaltenden Phase charakterisiert. Nach beendeter Hydroformylierungsreaktion läßt sich die wäßrige Katalysatorphase durch einfache Phasentrennung von der organischen Produktphase abtrennen und wieder in den Hydroformylierungsprozeß zurückführen.

Die phosphansubstituierten Cobalticiniumsalze der allgemeinen Formel I lassen sich als Diphosphane auffassen. Die Ligandeneigenschaften der Diphosphane gegenüber einem Metallzentrum werden u.a. nach Casey durch den sogenannten Natural Bite Angle bestimmt. Darunter versteht man eine Quantifizierung der konformationellen Beweglichkeit der Chelatliganden, bestimmte Koordinationsstellen an Metallatomen einzunehmen. Besonders bevorzugt ist ein Bißwinkel um 120°, da in dieser Anordnung an einer trigonalen Bipyramide eine diäquatoriale Position eingenommen werden kann.

Rhodium gelangt entweder als Metall oder als Verbindung zum Einsatz. In metallischer Form verwendet man es entweder als feinverteilte Partikel oder in dünner Schicht auf einem Träger, wie Aktivkohle, Calciumcarbonat, Aluminiumsilikat, Tonerde niedergeschlagen. Als Rhodiumverbindungen eignen sich Salze aliphatischer Mono- und Polycarbonsäuren, wie Rhodium-2-ethylhexanoat, Rhodiumacetat, Rhodiumoxalat, Rhodiumpropionat oder Rhodiummalonat. Weiterhin können Rhodiumsalze anorganischer Wasserstoff- und Sauerstoffsäuren, wie Rhodiumnitrat oder Rhodiumsulfat, die verschiedenen Rhodiumoxide oder auch Rhodiumcarbonylverbindungen wie Rh₃ (CO)₁₂ oder Rh₆(CO)₁₆ oder Komplexverbindungen des Rhodiums, z.B. Cyclooctadienyl-rhodiumverbindungen oder Rhodiumacetylacetonat, eingesetzt werden. Rhodiumhalogenverbindungen kommen wegen des korrosiven Verhaltens der Halogenidionen weniger in Betracht.

Bevorzugt werden Rhodiumoxid und insbesondere Rhodiumacetat und Rhodium-2-ethylhexanoat.

Der Katalysator kann dem Reaktionsgemisch präformiert zugesetzt werden. Dabei wird zu der Lösung des phosphansubstituierten Cobalticiniumsalzes der allgemeinen Formel I in einem organischen Lösungsmittel die gewünschte Menge Rhodium, vorzugsweise als Rhodium-2-ethylhexanoat-Lösung hinzugegeben und das Reaktionsgemisch bei einer Temperatur von 100 bis 120°C mit Synthesegas bei einem Druck von 0,5 bis 5 MPa über einen Zeitraum bis zu 5 Stunden behandelt. Nach der Präformierung des Katalysators erfolgt die Umsetzung der Olefine mit Wasserstoff und Kohlenmonoxid bei Temperaturen von 20 bis 150°C, bevorzugt 80 bis 140°C und insbesondere 100 bis 125°C und Drücken von 0,1 bis 20 MPa, bevorzugt 1 bis 12 MPa und insbesondere 3 bis 7 MPa.

Die Wahl des Lösungsmittels richtet sich dabei nach dem Lösungsverhalten des jeweiligen phosphansubstituierten Cobalticiniumsalzes in Abhängigkeit von dem Anion X. Vorzugsweise erfolgt die Präformierung in Toluol, Xylol, Tetrahydrofuran und insbesondere in Aceton als Lösungsmittel. Unter den phosphansubstituierten Cobalticiniumsalzen der allgemeinen Formel I sind besonders 1,1'-Bis(diphenylphosphino)cobalticinium Tetrafluoroborat, 1,1'-Bis(diphenylphosphino)cobalticinium Nitrat oder 1,1'-Bis(diphenylphosphino)cobalticinium Sulfat bevorzugt.

Die Zusammensetzung des Synthesegases, d.h. das Verhältnis von Kohlenmonoxid zu Wasserstoff, kann in weiten Grenzen variiert werden. Im allgemeinen setzt man Synthesegas ein, in dem das Volumenverhältnis von Kohlenmonoxid zu Wasserstoff 1:1 beträgt oder von diesem Wert nur wenig abweicht.

Mit gleich gutem Erfolg läßt sich der Katalysator aus dem phosphansubstituierten Cobalticiniumsalz und dem Rhodiummetall oder der Rhodiumverbindung unter Reaktionsbedingungen, also in Gegenwart des Olefins herstellen. Dabei kann das Olefin und der gebildete Aldehyd als Lösungsmittel für den Katalysator dienen. Arbeitet man mit einem Lösungsmittel, so wird bevorzugt Toluol, Xylol, Tetrahydrofuran und insbesondere Aceton verwendet.

Die Rhodiumkonzentration beträgt 2 bis 800 Gew.-ppm, vorzugsweise 2 bis 100 Gew.-ppm und insbesondere 5 bis 30 Gew.-ppm, bezogen auf die eingesetzte Olefinmenge.

Es hat sich bewährt Rhodium und das phosphansubstituierte Cobalticinium-Salz nicht in stöchiometrischem Verhältnis, also entsprechend der chemischen Zusammensetzung der Rhodium-Komplexverbindung zu verwenden, die sich im Verlauf der Hydroformylierungsreaktion bildet, sondern das phosphansubstituierte Cobalticinium Salz im Überschuß einzusetzen. Dabei kann man das Verhältnis von Rhodium zu dem phosphansubstituierten Cobalticinium Salz, auch ausgedrückt als das Verhältnis von Rhodium zu Phosphor (III), in weiten Grenzen variieren und je mol Rhodium etwa 2 bis 1000 mol Phosphor (III) anwenden. Bevorzugt wird ein Molverhältnis Phosphor(III) zu Rhodium von 3 bis 300 und insbesondere von 20 bis 100.

Die Umsetzung kann sowohl absatzweise als auch kontinuierlich durchgeführt werden. Nach erfolgter Hydroformylierungsreaktion werden die gewünschten Aldehyde durch Destillation von dem Katalysator und gegebenenfalls von dem Lösungsmittel abgetrennt.

Die folgenden Beispiele erläutern das erfindungsgemäße Verfahren, beschränken es jedoch nicht.

### Beispiel 1: Hydroformylierung in Gegenwart von 1,1'-Bis-(diphenylphosphino)cobalticinium Tetrafluoroborat.

In einem 250 Liter Kolben wurden zu 100 g Aceton 1,41 ml einer Rhodium-2-ethylhexanoat-Lösung in 2-Ethylhexanol (Rhodium-Konzentration: 4,26 g/l) und 1,87 g (2,9 mmol) 1,1'-Bis(diphenylphosphino)cobalticinium Tetrafluoroborat gegeben, entsprechend einem Molverhältnis Phosphor(III) zu Rhodium von 100. Unter Stickstoff wurde der Kolbeninhalt in einen mehrfach mit Stickstoff gespülten Autoklaven gedrückt. Danach leitete man zur Präformierung des Katalysators bei einer Temperatur von 120°C und 2,5 MPa unter Rühren Synthesegas (CO/H₂-Vol.-Verhältnis = 1 : 1) ein. Nach zwei Stunden wurde die Präformierung des Katalysators beendet und der Autoklav auf 20°C abgekühlt und bis auf einen Druck von 0,5 MPa entspannt. Anschließend wurden 300 g n-Hexen-1 bei einer Temperatur von 120°C und bei einem Synthesegasdruck von 1,5 MPa über einen Zeitraum von 1 Stunde in den Reaktor gepumpt. Danach wurde Synthesegas bei einer Temperatur von 120°C und bei einem Druck von 2,5 MPa in den Reaktor geleitet. Bezogen auf die eingesetzte Olefinmenge betrug der Rhodiumgehalt 20 ppm. Die Reaktion wurde nach drei Stunden abgebrochen, der Autoklav entspannt und das Reaktionsprodukt über einen Tauchstutzen entnommen und analysiert. Der Olefinumsatz beträgt 73 %, das Verhältnis von n-Heptanal zu 2-Methylhexanal beträgt 92 : 8.

### Beispiel 2: Hydroformylierung in Gegenwart von Triphenylphosphin (Vergleichsbeispiel)

Analog der in Beispiel 1 beschriebenen Arbeitsweise wurden in 100 g Aceton 1,41 ml einer Rhodium-2-ethylhexanoat-Lösung in 2-Ethylhexanol (Rhodium-Konzentration: 4,26 g/l) und 1,53 g (5,83 mmol) Triphenylphosphan gegeben, entsprechend einem Molverhältnis Phosphor(III) zu Rhodium von 100. Gemäß Beispiel 1 wurde der Katalysator präformiert. Nach erfolgter Präformierung wurden 300 g n-Hexen-1 in den Autoklaven gegeben und entsprechend Beispiel 1 mit Synthesegas umgesetzt. Der Rhodiumgehalt, bezogen auf die eingesetzte Olefinmenge, betrug 20 ppm. Nach Aufarbeitung gemäß Beispiel 1 ergab die Analyse des Reaktionsgemisches einen Olefinumsatz von 8 % bei einem Verhältnis von n-Heptanal zu 2-Methylhexanal von 74 : 26.

### Beispiel 3: Hydroformylierung in Gegenwart von 2,2'-Bis-(diphenylphosphinomethyl)-1,1'-binaphtyl (Vergleichsbeispiel)

Analog der in Beispiel 1 beschriebenen Arbeitsweise wurden in 100 g Aceton 1,41 ml einer Rhodium-2-ethylhexanoat-Lösung in 2-Ethylhexanol (Rhodium-Konzentration: 4,26 g/l) und 1,92 g (3 mmol) 2,2'-Bis-(diphenylphosphinomethyl)-1,1'-binaphtyl gegeben, entsprechend einem Molverhältnis Phosphor(III) zu Rhodium von 100. Nach erfolgter Präformierung gemäß Beispiel 1 wurden 300 g n-Hexen-1 in den Autoklaven gegeben und entsprechend Beispiel 1 mit Synthesegas umgesetzt. Der Rhodiumgehalt, bezogen auf die eingesetzte Olefinmenge, betrug 20 ppm.

Nach Aufarbeitung gemäß Beispiel 1 ergab die Analyse des Reaktionsgemisches einen Olefinumsatz von 77 % bei einem Verhältnis von n-Heptanal zu 2-Methylhexanal von 98 : 2.

Die Verwendung der erfindungsgemäßen Verbindungen als Liganden in Metallkomplexen führt im Vergleich zu der Verwendung von Triphenylphosphin bei Hydroformylierungsreaktionen von Olefinen zu deutlich höheren Olefinumsätzen und höheren Selektivitäten in Bezug auf die gewünschten geradkettigen Aldehyde. Im Vergleich zu dem bekannten, aber aufwendig herzustellenden Diphosphan 2,2'-Bis-(diphenylphosphinomethyl)-1,1'-binaphthyl (Vergleichsbeispiel 3) sind Olefinumsatz und Selektivität zu den gewünschten geradkettigen Aldehyden nur wenig schlechter.

### Beispiel 4:

Entsprechend der Arbeitsweise gemäß Beispiel 1 wurde eine Lösung von 1,1'-Bis(diphenylphosphino)cobalticinium Tetrafluoroborat in Aceton entsprechend einem Molverhältnis Phosphor(III) zu Rhodium von 20 mit einer Lösung von Rhodium-2-ethylhexanoat versetzt.

Die Präformierung des Katalysators erfolgte bei einem Druck von 2,5 MPa und bei einer Temperatur von 110°C mit Synthesegas (CO/H₂-Vol.-Verhältnis = 1 : 1) über einen Zeitraum von 0,5 Stunden. Anschließend wurde n-Hexen-1 in einer solcher Menge zugegeben, daß das Verhältnis n-Hexen-1 zu Aceton 1 : 5 und die Rhodiumkonzentration, bezogen auf die eingesetzte Olefinmenge, 800 ppm betrug. Die Umsetzung mit Synthesegas erfolgte bei einer Temperatur von 110°C und einem Druck von 2,5 MPa über einen Zeitraum von 0,5 Stunden. Der Olefinumsatz beträgt 99,7 %, das Verhältnis n-Heptanal zu 2-Methylhexanal 90 : 10.

### Beispiel 5: (Vergleichsbeispiel)

Analog der Arbeitsweise aus Beispiel 4 wurde in Toluol als Lösungsmittel durch Zugabe von 2,2'-Bis-(diphenylphosphinomethyl)-1,1'-binaphthyl und einer Rhodium-2-ethylhexanoat-Lösung ein Molverhältnis Phosphor(III) zu Rhodium von 20 eingestellt. Die Präformierung des Katalysators erfolgte bei einem Druck von 1,05 MPa und bei einer Temperatur von 110°C mit Synthesegas (CO : H₂-Vol.-Verhältnis 1 : 2) über einen Zeitraum von 25 Minuten. Anschließend wurde n-Hexen-1 in einer solchen Menge zugegeben, daß das Verhälntis n-Hexen-1 zu Toluol 1 : 5 und die Rhodiumkonzentration, bezogen auf die eingesetzte Olefinmenge, 800 ppm betrug. Die Umsetzung mit Synthesegas (CO : H₂-Vol.-Verhältnis 1 : 2) erfolgte bei einer Temperatur von 110°C und einem Druck von 1,05 MPa über einen Zeitraum von 25 Minuten. Der Olefinumsatz beträgt 97 %, das Verhältnis n-Heptanal zu 2-Methylhexanal 99 : 1.

Wie aus Beispiel 4 und 5 ersichtlich, lassen sich nach dem erfindungsgemäßen Verfahren bei erhöhtem Rhodiumeinsatz Olefinumsätze beobachten, die mit denen vergleichbar sind, die man bei der Verwendung aufwendig synthetisierter Diphosphane erhält.

### Beispiel 6:

Entsprechend der Arbeitsweise aus Beispiel 1 wurde eine Lösung von 1,1'-Bis(diphenylphosphino)cobalticinium Hexafluorophosphat in Aceton entsprechend einem Molverhältnis Phosphor (III) zu Rhodium von 30 mit einer Lösung von Rhodium-2-ethylhexanoat versetzt.

Die Präformierung des Katalysators erfolgte gemäß der Vorschrift nach Beispiel 1. Anschließend wurde n-Hexen-1 in einer solchen Menge zugegeben, daß das Verhältnis n-Hexen-1 zu Aceton 3 : 1 und die Rhodiumkonzentration, bezogen auf die eingesetzte Olefinmenge, 30 ppm betrug. Die Umsetzung mit Synthesegas erfolgte nach der Vorschrift von Beispiel 1 mit der einzigen Ausnahme, daß die Reaktionsdauer nunmehr vier Stunden betrug. Man erhält einen Olefinumsatz von 58 % und ein Verhältnis von n-Heptanal zu Methylhexanal von 76 : 24.

### Beispiel 7 :

Eine Lösung von 1,1'-Bis(diphenylphosphino)cobalticinium Nitrat in Wasser wurde entsprechend einem Molverhältnis Phosphor (III) zu Rhodium von 30 mit einer wäßrigen Lösung von Rhodiumacetylacetonat versetzt.

Die Präformierung des Katalysators erfolgte bei einem Druck von 2,5 MPa und bei einer Temperatur 125°C mit Synthesegas (CO/H₂ - Vol.-Verhältnis = 1 : 1) über einen Zeitraum von 1,5 Stunden. Anschließend erfolgte die n-Hexen-1 Zugabe in einer solchen Menge, daß die Rhodium-konzentration, bezogen auf auf die eingesetzte Olefinmenge, 30 ppm betrug. Die Umsetzung mit Synthesegas erfolgte bei einer Temperatur von 125°C und einem Druck von 2,5 MPa über einen Zeitraum von drei Stunden. Nach beendeter Reaktion wurde die organische Phase von der wäßrigen, katalysatorhaltigen Phase durch Phasentrennung separiert und analysiert. Der Olefinumsatz beträgt 17,8 % und das Verhältnis n-Heptanal zu 2-Methylhexanal 65: 35.

## Patentansprüche

1. Verfahren zur Herstellung von Aldehyden durch Umsetzung von Monoolefinen, nichtkonjugierten Polyolefinen, Cycloolefinen oder Derivaten dieser Verbindungsklasse mit Kohlenmonoxid und Wasserstoff bei Temperaturen von 20 bis 150°C und Drücken von 0,1 bis 20 MPa in Gegenwart einer organischen Lösung als Katalysator, die mindestens eine Rhodiumverbindung und mindestens ein phosphansubstituiertes Cobalticiniumsalz enthält, **dadurch gekennzeichnet, daß** phosphansubstituierte Cobalticiniumsalze der allgemeinen Formel I 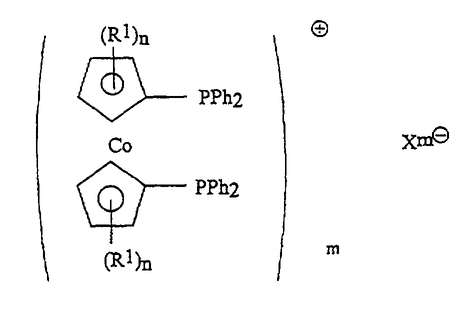 verwendet werden, wobei R¹ gleich oder verschieden ist und Wasserstoff, Alkyl- oder Alkoxyreste mit 1 bis 14 Kohlenstoffatomen, ferner Cycloalkyl-, Aryl-oder Aroxyreste mit 6 bis 14 Kohlenstoffatomen, einen anellierten Benzolring, einen Säureamidrest -C(O) -NR²R³, in dem R² und R³ gleich oder verschieden sind und für Wasserstoff, Alkylreste mit 1 bis 14 Kohlenstoffatomen, Cycloalkyl- oder Arylreste mit 6 bis 14 Kohlenstoffatomen stehen, die Carboxylgruppe oder einen Esterrest -C(O)-OR⁴, in dem R⁴ für einen Alkylrest mit 1 bis 14 Kohlenstoffatomen, ferner für einen Cycloalkyl- oder Arylrest mit 6 bis 14 Kohlenstoffatomen steht, bedeutet, n gleich oder verschieden ist und eine ganze Zahl von 0 bis 4 ist, m eine ganze Zahl von 1 bis 3 ist und X gleich Hexafluorophosphat, Hexafluoroantimonat, Tetraphenylborat, Tosylat, Tetrafluoroborat, Triflat, Trifluoracetat, Triiodid, Tribromid, Nitrat, Perchlorat, Sulfat, Phosphat, Sulfonat ⁻O-S(O)₂-R⁵, wobei R⁵ einen Alkylrest mit 1 bis 14 Kohlenstoffatomen, Cycloalkyl-oder Arylrest mit 6 bis 14 Kohlenstoffatomen bedeutet, oder Carboxylat ⁻O-C(O)-R⁶, wobei R⁶ für Wasserstoff, einen Alkylrest mit 1 bis 14 Kohlenstoffatomen, ferner für einen Cycloalkyl- oder Arylrest mit 6 bis 14 Kohlenstoffatomen steht, ist.

2. Verfahren nach Anspruch 1, **dadurch gekennzeichnet, daß** in der allgemeinen Formel I R¹ für Wasserstoff, für einen Methyl-, Isopropyl-, Isobutyl-, t-Butyl, Phenyl-, Naphthylrest oder einen anellierten Benzolring steht.

3. Verfahren nach einem der Ansprüche 1 und 2, **dadurch gekennzeichnet, daß** in der allgemeinen Formel I n gleich 0 oder 1 ist.

4. Verfahren nach einem der Ansprüche 1 bis 3, **dadurch gekennzeichnet, daß** in der allgemeinen Formel m gleich 1 oder 2 ist.

5. Verfahren nach einem der Ansprüche 1 bis 4, **dadurch gekennzeichnet, daß** in der allgemeinen Formel X für Tetrafluoroborat, Nitrat oder Sulfat steht.

6. Verfahren zur Herstellung von Aldehyden durch Umsetzung von Monoolefinen, nichtkonjugierten Polyolefinen, Cycloolefinen oder Derivaten dieser Verbindungsklasse mit Kohlenmonoxid und Wasserstoff bei Temperaturen von 20 bis 150°C und Drücken von 0,1 bis 20 MPa in Gegenwart einer wäßrigen, mindestens eine Rhodiumverbindung enthaltende Lösung als Katalysator, **dadurch gekennzeichnet, daß** die wäßrige Lösung 1,1'-Bis(diphenylphosphino)cobalticinium Nitrat enthält.

7. Verfahren nach einem der Ansprüche 1 bis 6, **dadurch gekennzeichnet, daß** je mol Rhodium 2 bis 1000 mol Phosphor(III) eingesetzt werden.

8. Verfahren nach Anspruch 7, **dadurch gekennzeichnet, daß** je mol Rhodium 3 bis 300 mol Phosphor(III) eingesetzt werden.

9. Verfahren nach Anspruch 8, **dadurch gekennzeichnet, daß** je mol Rhodium 20 bis 100 mol Phosphor(III) eingesetzt werden.

10. Verfahren nach einem der Ansprüche 1 bis 9, **dadurch gekennzeichnet, daß** die Rhodiumkonzentration 2 bis 800 Gew.-ppm, bezogen auf die eingesetzte Olefinmenge, beträgt.

11. Verfahren nach Anspruch 10, **dadurch gekennzeichnet, daß** die Rhodiumkonzentration 2 bis 100 Gew.-ppm, bezogen auf die eingesetzte Olefinmenge, beträgt.

12. Verfahren nach Anspruch 11, **dadurch gekennzeichnet, daß** die Rhodiumkonzentration 5 bis 30 Gew.-ppm, bezogen auf die eingesetzte Olefinmenge, beträgt.

13. Verfahren nach einem der Ansprüche 1 bis 12, **dadurch gekennzeichnet, daß** die Umsetzung bei 20 bis 150°C erfolgt.

14. Verfahren nach Anspruch 13, **dadurch gekennzeichnet, daß** die Umsetzung bei 80 bis 140°C erfolgt.

15. Verfahren nach Anspruch 14, **dadurch gekennzeichnet, daß** die Umsetzung bei 100 bis 125°C erfolgt.

16. Verfahren nach einem der Ansprüche 1 bis 15, **dadurch gekennzeichnet, daß** die Umsetzung bei Drücken von 0,1 bis 20 MPa erfolgt.

17. Verfahren nach Anspruch 16, **dadurch gekennzeichnet, daß** die Umsetzung bei Drücken von 1 bis 12 MPa erfolgt.

18. Verfahren nach Anspruch 17, **dadurch gekennzeichnet, daß** die Umseetzung bei Drücken von 3 bis 7 MPa erfolgt.

19. Verfahren nach einem der Ansprüche 1 bis 18, **dadurch gekennzeichnet, daß** Olefine oder Olefinderivate mit 2 bis 12 Kohlenstoffatomen eingesetzt werden.

20. Verfahren zur Herstellung von Aldehyden nach Anspruch 6, **dadurch gekennzeichnet, daß** nach der Hydroformylierungsreaktion die Abtrennung der wäßrigen Katalysatorlösung durch Phasentrennung erfolgt.

21. Katalysator zur Hydroformylierung von Monoolefinen, nichtkonjugierten Polyolefinen, Cycloolefinen oder Derivaten dieser Verbindungsklasse enthaltend Rhodium und ein phosphansubstituiertes Cobalticinium-salz, **dadurch gekennzeichnet, daß** das phosphansubstituierte Cobalticiniumsalz der allgemeinen Formel I 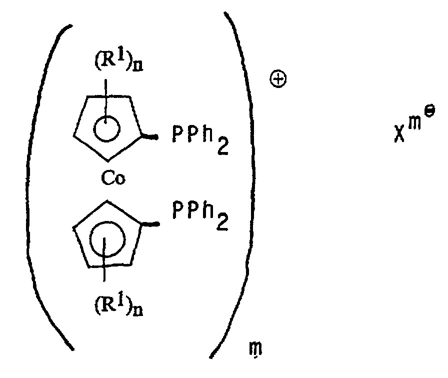 aufweist, wobei R¹ gleich oder verschieden ist und Wasserstoff, Alkyl- oder Alkoxyreste mit 1 bis 14 Kohlenstoffatomen, ferner Cycloalkyl-, Aryl- oder Aroxyreste mit 6 bis 14 Kohlenstoffatomen, einen anellierten Benzolring, einen Säureamidrest -C(O)-NR²R³, in dem R² und R³ gleich oder verschieden sind und für Wasserstoff, Alkylreste mit 1 bis 14 Kohlenstoffatomen, Cycloalkyl- oder Arylreste mit 6 bis 14 Kohlenstoffatomen stehen, die Carboxylgruppe oder einen Esterrest -C(O)-OR⁴, in dem R⁴ für einen Alkylrest mit 1 bis 14 Kohlenstoffatomen, ferner für einen Cycloalkyl- oder Arylrest mit 6 bis 14 Kohlenstoffatomen steht, bedeutet, n gleich oder verschieden ist und eine ganze Zahl von 0 bis 4 ist, m eine ganze Zahl von 1 bis 3 ist, und wobei das Anion der allgemeinen Formel X gleich Hexafluoroantimonat, Tetraphenylborat, Tosylat, Tetrafluorcborat, Triflat, Trifluoracetat, Triiodid, Tribromid, Nitrat, Perchlorat, Sulfat, Phosphat, Sulfonat ⁻O-S(O)₂-R⁵, wobei R⁵ einen Alkylrest mit 1 bis 14 Kohlenstoffatomen, Cycloalkyl- oder Arylrest mit 6 bis 14 Kohlenstoffatomen bedeutet, oder Carboxylat ⁻O-C(O)-R⁶, wobei R⁶ für Wasserstoff, einen Alkylrest mit 1 bis 14 Kohlenstoffatomen, ferner für einen Cycloalkyl- oder Arylrest mit 6 bis 14 Kohlenstoffatomen steht, zugegen ist.

## Claims

1. A process for the preparation of aldehydes by reacting monoolefins, unconjugated polyolefins, cycloolefins or derivatives of this class of compounds, with carbon monoxide and hydrogen at temperatures of 20 to 150°C and pressures of 0.1 to 20 MPa in the presence as catalyst of an organic solution which comprises at least one rhodium compound and at least one phosphine-substituted cobalticinium salt, which comprises using phosphine-substituted cobalticinium salts of the formula I 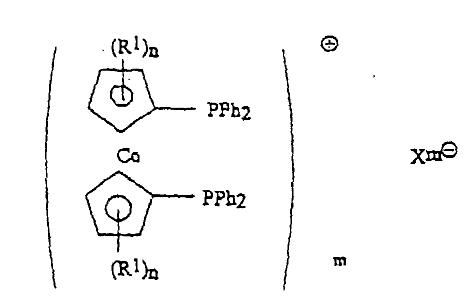 where R¹ is identical or different and is hydrogen; alkyl or alkoxy radicals having 1 to 14 carbon atoms; in addition cycloalkyl, aryl or aroxy radicals having 6 to 14 carbon atoms; a fused benzene ring; an amide radical -C(O)-NR²R³, in which R² and R³ are identical or different and are hydrogen, alkyl radicals having 1 to 14 carbon atoms, cycloalkyl or aryl radicals having 6 to 14 carbon atoms; the carboxyl group or an ester radical -C(O)-OR⁴, in which R⁴ is an alkyl radical having 1 to 14 carbon atoms, or in addition a cycloalkyl or aryl radical having 6 to 14 carbon atoms; n is identical of different and is an integer from 0 to 4; m is an integer from 1 to 3; and X is hexafluorophosphate, hexafluoroantimonate, tetraphenylborate, tosylate, tetrafluoroborate, triflate, trifluoroacetate, triiodide, tribromide, nitrate, perchlorate, sulfate, phosphate, sulfonate ⁻O-S(O)₂-R⁵, where R⁵ is an alkyl radical having 1 to 14 carbon atoms, a cycloalkyl or aryl radical having 6 to 14 carbon atoms, or carboxylate ⁻O-C(O)-R⁶, where R⁶ is hydrogen, an alkyl radical having 1 to 14 carbon atoms or additionally a cycloalkyl or aryl radical having 6 to 14 carbon atoms.

2. The process as claimed in claim 1, wherein, in the formular I, R¹ is hydrogen, a methyl, isopropyl, isobutyl, t-butyl, phenyl, or naphthyl radical or a fused benzene ring.

3. The process as claimed in one of claims 1 and 2, wherein, in the formula I, n is 0 or 1.

4. The process as claimed in one of claims 1 to 3, wherein, in the formula, m is 1 or 2.

5. The process as claimed in one of claims 1 to 4, wherein, in the formula, X is tetrafluoroborate, nitrate or sulfate.

6. A process for the preparation of aldehydes by reacting monoolefins, unconjugated polyolefins, cycloolefins or derivatives of this class of compounds, with carbon monoxide and hydrogen at temperatures of 20 to 150°C and pressures of 0.1 to 20 MPa in the presence as catalyst of an aqueous solution which comprises, that the aqueous solution contain at least one rhodium compound and 1,1'-bis(diphenylphosphino)-cobalticinium nitrate.

7. The process as claimed in one of claims 1 to 6, wherein 2 to 1000 mol of phosphorus(III) are used per mol rhodium.

8. The process as claimed in claim 7, wherein 3 to 300 mol of phosphorus(III) are used per mol rhodium.

9. The process as claimed in claim 8, wherein 20 to 100 mol of phosphorus(III) are used per mol rhodium.

10. The process as claimed in one of claims 1 to 9, wherein the rhodium concentration is 2 to 800 ppm by weight, based on the amount of olefin used.

11. The process as claimed in claim 10, wherein the rhodium concentration is 2 to 100 ppm by weight, based on the amount of olefin used.

12. The process as claimed in claim 11, wherein the rhodium concentration is 5 to 30 ppm by weight, based on the amount of olefin used.

13. The process as claimed in one of claims 1 to 12, wherein the reaction is carried out at 20 to 150°C.

14. The process as claimed in claim 13, wherein the reaction is carried out at 80 to 140°C.

15. The process as claimed in claim 14, wherein the reaction is carried out at 100 to 125°C.

16. The process as claimed in one of claims 1 to 15, wherein the reaction is carried out at pressures of 0.1 to 20 MPa.

17. The process as claimed in claim 16, wherein the reaction is carried out at pressures of 1 to 12 MPa.

18. The process as claimed in claim 17, wherein the reaction is carried out at pressures of 3 to 7 MPa.

19. The process as claimed in one of claims 1 to 18, wherein use is made of olefins or olefin derivatives having 2 to 12 carbon atoms.

20. The process for the preparation of aldehydes as claimed in claim 6, wherein, after the hydroformylation reaction, the aqueous catalyst solution is separated off by phase separation.

21. A catalyst for the hydroformylation of monoolefins, unconjugated polyolefins, cycloolefins or derivatives of this class of compounds which comprises rhodium and a phosphine-substituted cobalticinium salt, wherein the phosphine-substituted cobalticinium salt has the fomula I 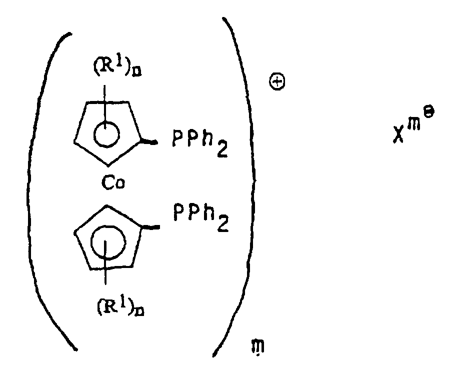 where R¹ is identical or different and is hydrogen; alkyl or alkoxy radicals having 1 to 14 carbon atoms; in addition cycloalkyl, aryl or aroxy radicals having 6 to 14 carbon atoms; a fused benzene ring; an amide radical -C(O)-NR²R³, in which R² and R³ are identical or different and are hydrogen, alkyl radicals having 1 to 14 carbon atoms, cycloalkyl or aryl radicals having 6 to 14 carbon atoms; the carboxyl group or an ester radical -C(O)-OR⁴, in which R⁴ is an alkyl radical having 1 to 14 carbon atoms, or in addition a cycloalkyl or aryl radical having 6 to 14 carbon atoms; n is identical or different and is an integer from 0 to 4, m is an integer from 1 to 3 and where the anion of the formula X is hexafluoroantimonate, tetraphenylborate, tosylate, tetrafluoroborate, triflate, trifluoroacetate, triiodide, tribromide, nitrate, perchlorate, sulfate, phosphate, sulfonate ⁻O-S(O)₂-R⁵, where R⁵ is an alkyl radical having 1 to 14 carbon atoms, a cycloalkyl or aryl radical having 6 to 14 carbon atoms, or carboxylate -O-C(O)-R⁶, where R⁶ is hydrogen, an alkyl radical having 1 to 14 carbon atoms or additionally a cycloalkyl or aryl radical having 6 to 14 carbon atoms.

## Revendications

1. Procédé de production d'aldéhydes par réaction de monooléfines, de polyoléfines non conjuguées, de cyclooléfines ou de dérivés de cette classe de composés avec le monoxyde de carbone et l'hydrogène à des températures de 20 à 150°C et des pressions de 0,1 à 20 MPa en présence d'une solution organique comme catalyseur qui contient au moins un composé du rhodium et au moins un sel de cobalticinium phosphane-substitué, **caractérisé en ce que** des sels de cobalticinium phosphane-substitués de formule générale I 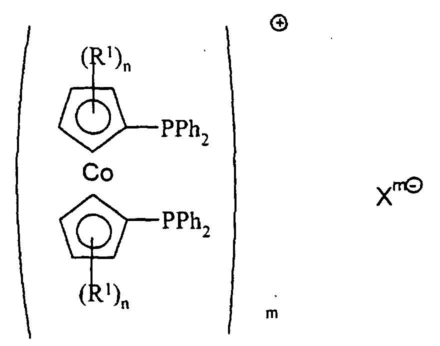 sont utilisés, où les R' sont identiques ou différents et représentent l'hydrogène, des restes alkyle ou alcoxy de 1 à 14 atomes de carbone, en outre des restes cycloalkyle, aryle ou aryloxy de 6 à 14 atomes de carbone, un cycle benzénique condensé, un reste amide d'acide -C(O)-NR²R³ où R² et R³ sont identiques ou différents et représentent l'hydrogène, des restes alkyle de 1 à 14 atomes de carbone, des restes cycloalkyle ou aryle de 6 à 14 atomes de carbone, le groupe carboxyle ou un reste ester -C(O)-OR⁴ où R⁴ représente un reste alkyle de 1 à 14 atomes de carbone, en outre un reste cycloalkyle ou aryle de 6 à 14 atomes de carbone, les n sont identiques ou différents et sont un nombre entier de 0 à 4, m est un nombre entier de 1 à 3 et X représente hexafluorophosphate, hexafluoroantimonate, tétraphénylborate, tosylate, tétrafluoroborate, triflate, trifluoroacétate, triiodure, tribromure, nitrate, perchlorate, sulfate, phosphate, sulfonate ⁻O-S(O)₂-R⁵, où R⁵ représente un reste alkyle de 1 à 14 atomes de carbone, un reste cycloalkyle ou aryle de 6 à 14 atomes de carbone, ou bien carboxylate ⁻O-C(O)-R⁶, où R⁶ représente l'hydrogène, un reste alkyle de 1 à 14 atomes de carbone, en outre un reste cycloalkyle ou aryle de 6 à 14 atomes de carbone.

2. Procédé selon la revendication 1, **caractérisé en ce que**, dans la formule générale I, R¹ représente l'hydrogène, un reste méthyle, isopropyle, isobutyle, t-butyle, phényle, naphtyle ou un cycle benzénique condensé.

3. Procédé selon l'une des revendications 1 et 2, **caractérisé en ce que**, dans la formule générale I, n est égal à 0 ou 1.

4. Procédé selon l'une des revendications 1 à 3, **caractérisé en ce que**, dans la formule générale, m est égal à 1 ou 2.

5. Procédé selon l'une des revendications 1 à 4, **caractérisé en ce que**, dans la formule générale, X représente tétrafluoroborate, nitrate ou sulfate.

6. Procédé de production d'aldéhydes par réaction de monooléfines, de polyoléfines non conjuguées, de cyclooléfines ou de dérivés de cette classe de composés avec le monoxyde de carbone et l'hydrogène à des températures de 20 à 150°C et des pressions de 0,1 à 20 MPa en présence d'une solution aqueuse contenant au moins un composé du rhodium comme catalyseur, **caractérisé en ce que** la solution aqueuse contient du nitrate de 1,1'-bis(diphénylphosphino)-cobalticinium.

7. Procédé selon l'une des revendications 1 à 6, **caractérisé en ce que** 2 à 1000 mol de phosphore (III) sont utilisées par mole de rhodium.

8. Procédé selon la revendication 7, **caractérisé en ce que** 3 à 300 mol de phosphore (III) sont utilisées par mole de rhodium.

9. Procédé selon la revendication 8, **caractérisé en ce que** 20 à 100 mol de phosphore (III) sont utilisées par mole de rhodium.

10. Procédé selon l'une des revendications 1 à 9, **caractérisé en ce que** la concentration du rhodium est de 2 à 800 ppm en masse par rapport à la quantité d'oléfine utilisée.

11. Procédé selon la revendication 10, **caractérisé en ce que** la concentration du rhodium est de 2 à 100 ppm en masse par rapport à la quantité d'oléfine utilisée.

12. Procédé selon la revendication 11, **caractérisé en ce que** la concentration du rhodium est de 5 à 30 ppm en masse par rapport à la quantité d'oléfine utilisée.

13. Procédé selon l'une des revendications 1 à 12, **caractérisé en ce que** la réaction a lieu à 20 à 150°C.

14. Procédé selon la revendication 13, **caractérisé en ce que** la réaction a lieu à 80 à 140°C.

15. Procédé selon la revendication 14, **caractérisé en ce que** la réaction a lieu à 100 à 125°C.

16. Procédé selon l'une des revendications 1 à 15, **caractérisé en ce que** la réaction a lieu à des pressions de 0,1 à 20 MPa.

17. Procédé selon la revendication 16, **caractérisé en ce que** la réaction a lieu à des pressions de 1 à 12 MPa.

18. Procédé selon la revendication 17, **caractérisé en ce que** la réaction a lieu à des pressions de 3 à 7 MPa.

19. Procédé selon l'une des revendications 1 à 18, **caractérisé en ce que** l'on utilise des oléfines ou des dérivés d'oléfine ayant 2 à 12 atomes de carbone.

20. Procédé de préparation d'aldéhydes selon la revendication 6, **caractérisé en ce que** la séparation de la solution aqueuse de catalyseur a lieu par séparation de phases après la réaction d'hydroformylation.

21. Catalyseur pour l'hydroformylation de monooléfines, de polyoléfines non conjuguées, de cyclooléfines ou de dérivés de cette classe de composés contenant du rhodium et un sel de cobalticinium phosphane-substitué, **caractérisé en ce que** le sel de cobalticinium phosphane-substitué présente la formule générale I 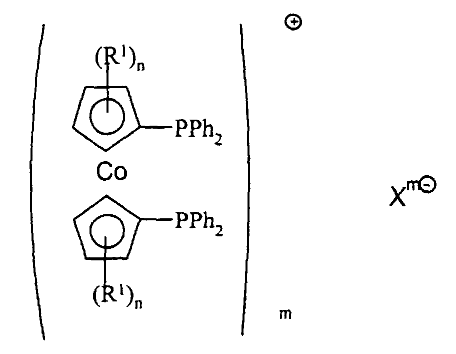 où les R¹ sont identiques ou différents et représentent l'hydrogène, des restes alkyle ou alcoxy de 1 à 14 atomes de carbone, en outre des restes cycloalkyle, aryle ou aryloxy de 6 à 14 atomes de carbone, un cycle benzénique condensé, un reste amide d'acide -C(O)-NR²R³ où R² et R³ sont identiques ou différents et représentent l'hydrogène, des restes alkyle de 1 à 14 atomes de carbone, des restes cycloalkyle ou aryle de 6 à 14 atomes de carbone, le groupe carboxyle ou un reste ester -C(O)-OR⁴ où R⁴ représente un reste alkyle de 1 à 14 atomes de carbone, en outre un reste cycloalkyle ou aryle de 6 à 14 atomes de carbone, les n sont identiques ou différents et sont un nombre entier de 0 à 4, m est un nombre entier de 1 à 3 et où l'anion de formule générale X représente hexafluoroantimonate, tétraphénylborate, tosylate, tétrafluoroborate, triflate, trifluoroacétate, triiodure, tribromure, nitrate, perchlorate, sulfate, phosphate, sulfonate ⁻O-S(O)₂-R⁵, où R⁵ représente un reste alkyle de 1 à 14 atomes de carbone, un reste cycloalkyle ou aryle de 6 à 14 atomes de carbone, ou bien carboxylate O-C(O)-R⁶, où R⁶ représente l'hydrogène, un reste alkyle de 1 à 14 atomes de carbone, en outre un reste cycloalkyle ou aryle de 6 à 14 atomes de carbone.
